# EUROPÄISCHE PATENTSCHRIFT

(11) **EP 3 308 836 B1**
(45) Veröffentlichungstag und Bekanntmachung des Hinweises auf die Patenterteilung: **09.09.2020**
(21) Anmeldenummer: 17199856.0
(22) Anmeldetag: 04.06.2014
(51) Int. Cl.: A61Q 17/04, A61K 8/29, A61K 8/49, A61K 8/31

(54) **OCTOCRYLENFREIES, GERUCHSSTABILES SONNENSCHUTZMITTEL**
OCTOCRYLENE-FREE, ODOUR-STABLE SUN PROTECTION AGENT
PRODUIT DE PROTECTION CONTRE LE SOLEIL SANS OCTOCRILÈNE AYANT UNE ODEUR STABLE

(30) Priorität: 04.07.2013 DE 102013213170
(43) Veröffentlichungstag der Anmeldung: 18.04.2018
(62) Teilanmeldung aus: 14171055.8
(73) Patentinhaber: Beiersdorf AG, 20253 Hamburg (DE)
(72) Erfinder: SKUBSCH, Kerstin, 25497 Prisdorf (DE); NIELSEN, Jens, 22529 Hamburg (DE)

(56) Entgegenhaltungen:
- EP-A1- 1 923 045
- WO-A2-2009/124632
- DE-A1- 10 155 957
- US-A1- 2005 008 587

## Beschreibung

Die vorliegende Erfindung betrifft eine kosmetische Zubereitung enthaltend ein oder mehrere UV-Filter aus der Gruppe der Triazinderivate, Titandioxid, 4-(tert.-Butyl)-4'-methoxydibenzoylmethan und/oder 2-(4'-(Diethylamino)-2'-hydoxybenzoyl)-benzoesäurehexylester, die dadurch gekennzeichnet ist, dass die Zubereitung frei ist von Ethylhexylsalicylat, 2-Ethylhexyl-2-cyano-3,3-diphenylacrylat, Homomenthylsalicylat und 4-Methoxyzimtsäure(2-ethylhexyl)ester.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Nachteilig am Stande der Technik ist insbesondere der Umstand, dass eine Reihe von Inhaltsstoffen in Sonnenschutzmittein photolabil sind und/oder unter thermischer Belastung zerstört werden oder sich verflüchtigen. Da Sonnenschutsmittel jedoch ihrem Zweck entsprechend stacker UV-Strahlung und Wärme ausgesetzt sind, ist die Thermo- und Photostabilität eine wichtige Voraussetzung für die Wirksamkeit während der gesamten Anwendungsdauer. Problematisch am Stande der Technik ist insbesondere, dass die Zubereitungen aufgrund dieser Phänomene mit zunehmender Anwendungsdauer ihren Geruch ändern. Der frische Duft zu Beginn der Anwendung weicht dann mit zunehmender Anwendungsdauer einem muffigen, ranzigen oder nach "Maggi"-Würze riechenden Duft. Ein solcher "Duftwandel" führt dann dazu, dass die Verbraucher den Einsatz von Sonnenschutzmittetn eher meiden.

Es war daher die Aufgabe der vorliegenden Erfindung, den Nachteil des Standes der Technik zu beseitigen und ein geruchsstabiles Sonnenschutzmittel zu entwickeln.

Ein weiterer Nachteil des Standes der Technik besteht in dem Umstand, dass der Einsatz einiger UV-Filter, trotz ihrer Zulassung durch die Genehmigungsbehörden, nicht ganz unumstritten ist und bei Bewertungen bei einigen Verbrauchermagazinen (z.B. Öko-Test) zu "Abwertungen" in der Benotung des Produktes führen. Dies betrifft beisplelsweise die UV-Filter Octocrylen, Homosalate oder Ethylhexyl-Methoxycinnamat, von denen einige Wissenschaftler vermuten, dass sie möglicherweise hormoneil wirksam sein könnten. Auch wenn, trotz des jahrzehntelangen weltweiten Einsatzes dieser UV-Filter in Sonnenschutzmitteln keine negativen Wirkungen am Menschen bekannt geworden sind, besteht bei den Verbrauchern der Wunsch, Zubereitungen mit derartigen inhaltsstoffen zu meiden.

Es war daher die Aufgabe der vorliegenden Erfindung, ein stabiles und wirksames Sonnenschutzmittel zu entwickeln, welches frei ist von Ethylhexylsalicylat, 2-Ethylhexyl-2-cyano-3,3-diphenylacrylat (Octocrylen), Homomenthylsalicylat (Homosalate) und 4-Methoxyzimtsäure(2-ethylhexyl)ester (Ethylhexyl Methoxycinnamate).

Überraschend gelöst werden die Aufgaben durch eine kosmetische Zubereitung gemäß Anspruch 1.

Für den Fachmann war dabei insbesondere nicht vorhersehbar, dass durch den Verzicht auf insbesondere 2-Ethylhexyl-2-cyano-3,3-diphenylacrylat (Octocrylen), aber auch auf Ethylhexylsalicylat, Homomenthytsalicylat und 4-Methoxyzimtsäure(2-ethylhexyl)ester die Zubereitung eine höhere Geruchsstabilität aufwies, als Zubereitungen mit diesen Filtern, denn eigentlich wäre zu erwarten gewesen, dass diese Stoffe aufgrund ihrer Filtereigenschaften die Zubereitungen geruchlich stabilisieren würden.

Zwar kennt der Stand der Technik die WO 2009/124632 A2, US 2005/008587 A1 und EP 1923045 A1, doch konnten diese Schriften nicht den Weg zur vorliegenden Erfindung weisen.

Es ist erfindungsgemäß von Vorteil, wenn die erfindungsgemäße Zubereitung frei ist von Polyethylenglycol, Polyethylenglycolethern und Polyethylenglycolestern (sogenannten PEG-Derivaten).

Es ist erfindungsgemäß vorteilhaft, wenn die erfindungsgemäße Zubereitung UV-Filter aus der Gruppe der Triazinderivate in einer Gesamtmenge von 0,1 bis 10 Gewichts-%, bezogen auf das Gesamtgewicht der Zubereitung, enthält.

Erfindungsgemäß vorteilhafte Ausführungsformen der vorliegenden Erfindung sind dadurch gekennzeichnet, dass die UV-Filter aus der Gruppe der Triazinderivate gewählt werden aus der Gruppe der Verbindungen Dioctylbutylamidotriazon (INCI: Diethylhexyl-Butamidotriazone); 2,4-Bis-{[4-(2-ethyl-hexyloxy)-2-hydroxy]-phenyl}~6~(4-methoxyphenyl)-1,3,5-triazin; 4,4',4"-(1,3,5-Triazin-2)4(6-triyltriimino)-tris-benzoësäure-tris(2-ethylhexylester)(auch:2,4,6-Tris-[anilino-(p-carbo-2'-ethyl-1'-hexyloxy}]-1,3,5-triazin (INCI: Ethylhexyl Triazone); 2,4,6-Tribiphenyl-4-yl-1,3,5-triazin.

Erfindungsgemäß bevorzugt sind dabei die Triazinderivate 2,4-Bis-{[4-(2-ethyl-hexyloxy)-2-hydroxy]-phenyl}-6-(4-methoxyphenyl)-1,3,5-triazin und 4,4',4"-(1,3,5-Triazin-2,4,6-triyltriimino)-tris-benzoësäure-tris(2-ethylhexylester).

Es ist erfindungsgemäß vorteilhaft, wenn die erfindungsgemäße Zubereitung 2,4-Bis-([4-(2-ethyl-hexyioxy)-2-hydroxyl-phonyl)-6-(4-methoxyphenyl)-1,3,5-triazin in einer Konzentration von 0,1 bis 5 Gewichts-%, bezogen auf das Gesamtgewicht der Zubereitung enthält.

Es ist erfindungsgemäß bevorzugt, wenn die erfindungsgemäße Zubereitung 2,4-Bis-{[4-(2-ethyl-hexyloxy)-2-hydroxy]-phenyl}-6-(4-methoxyphenyl)-1,3,5-triazin in einer Konzentration von 0,5 bis 4 Gewichts-%, bezogen auf das Gesamtgewicht der Zubereitung enthält.

Es ist erfindungsgemäß vorteilhaft, wenn die erfindungsgemäße Zubereitung 4,4',4"-(1,3,5-Triazin-2,4,6-triyltriimino)-tris-benzoesäure-tris(2-ethylhexylester) in einer Konzentration von 0,1 bis 6 Gewichts-%, bezogen auf das Gesamtgewicht der Zubereitung enthält.

Es ist erfindungsgemäß bevorzugt, wenn die erfindungsgemäße Zubereitung 4,4',4"-(1,3,5-Triazin-2,4,6-triyltriimino)-tris-benzoesäure-tris(2-ethylhexylester) in einer Konzentration von 0,5 bis 5 Gewichts-%, bezogen auf das Gesamtgewicht der Zubereitung enthält.

Erfindungsgemäß vorteilhafte Ausführungsformen der vorliegenden Erfindung sind dadurch gekennzeichnet, dass die Zubereitung ein oder mehrere Öle gewählt aus der Gruppe der Verbindungen Butylene Glycol Dicaprylat/Dicaprat, Phenethyl Benzoat, C12-15 Alkyl Benzoat, Dibutyladipat; Diisopropylsebacate, Di-C12-13 Alkyl Tartrate, Butyloctyl Salicylate, Diethylhexyl Syringylidene Malonate, Hydragenated Castor Oil Dimerate, Triheptanoin, C12-13 Alkyl Lactate, C16-17 Alkyl Benzoate, Propylheptyl Caprylate, Caprylic/Capric Triglyceride, Diethylhexyl 2,6-Naphthalate, Octyldodecanol, Caprylic/Capric Triglyceride, Ethylhexyl Cocoate, enthält.

Es ist erfindungsgemäß vorteilhaft, diese Öle in einer Gesamtkonzentration von 5 bis 30 Gewichts-%, bezogen auf das Gesamtgewicht der Zubereitung einzusetzen.

Es hat sich in diesem Zusammenhang als für den Fachmann überraschend und nicht vorhersehbar herausgestellt, dass sich mit diesen Ölen auch unter Verzicht auf die erfindungsgemäß ausgeschlossenen flüssigen UV-Filter Ethylhexylsalicylat, 2-Ethylhexyl-2-cyano-3,3-diphenylacrylat, Homomenthylsalicylat und 4-Methoxyzimtsäure(2-ethylhexyl)ester, die Triazinderivate in ausreichender Form lösen und in die erfindungsgemäße Zubereitung einarbeiten lassen.

Das erfindungsgemäße Titandioxid liegt erfindungsgemäß in partikulärer Form vor.

Es ist erfindugnsgemäß vorteilhaft, wenn das Titandioxid eine Primärpartikelgröße von kleiner als 100 nm aufweist.

Erfindungsgemäß bevorzugt haben die Primärpartikel des erfindungsgemäßen Titandioxids eine Größe von 10 bis 80 nm (Dieser Wert wurde mittels Transmissionselektronenmikroskopie ermittelt.).

Unter Primärpartikeln können erfindungsgemäß die Partikel im Quellenzustand verstanden werden, d. h. in geringem zeitlichen und räumlichen Abstand von der Quelle. Das heißt in der strengen Definition nur unmittelbar nach der Nukleation und vor dem Einsetzen von Agglomerationsvorgängen. Die Wert der Primärpartikel können mit verschiedenen Methoden ermittelt werden: Erfindungsgemäß bevorzugt ist die Bestimmung mittels Transmissionselektronenmikroskopie.

Es ist erfindungsgemäß vorteilhaft, wenn das erfindungsgemäße Titandioxid oberflächenbeschichtet ist

Erfindungsgemäß bevorzugte Ausführungsformen sind insbesondere dadurch gekennzeichnet, dass die Titandioxid-Partikel mit Dimethicone/Methicon-Copolymer, Dimethoxycaprylylsilan, Aluminium Hydroxid und/oder Silica und/oder Stearinsäure oberflächenbeschichtet sind.

Vorteilhafte Ausführungsformen der vorliegenden Erfindung sind dadurch gekennzeichnet, dass die Zubereitung zwei unterschiedliche Titandioxide enthält, die sich in ihrer Primärpartikelgröße voneinander unterscheiden.

In einer erfindungsgemäß besonderes bevorzugten Ausführungsform der vorliegenden Erfindung werden zwei unterschiedliche Titandioxid Sorten eingesetzt, bei denen ein Teil der Titandioxid-Partikel (A) mit Alumina und Stearinsäure oberflächenbeschichtet ist und ein anderer Teil (B) mit Dimethoxycaprylylsilan oberflächenbeschichtet ist.

Es ist erfindungsgemäß vorteilhaft, wenn die erfindungsgemäße Zubereitung Titandioxid in einer Gesamtmenge von 0,5 bis 20 Gewichts-%, bezogen auf das Gesamtgewicht der Zubereitung, enthält.

Es ist erfindungsgemäß bevorzugt, wenn die erfindungsgemäße Zubereitung Titandioxid in einer Gesamtmenge von 1 bis 7 Gewichts-%, bezogen auf das Gesamtgewicht der Zubereitung, enthält.

Erfindungsgemäß enthält die erfindungsgemäße Zubereitung 4-(tert.-Butyl)-4'-methoxydibenzoylmethan und/oder 2-(4'-(Diethylamino)-2'-hydoxybenzoyl)-benzoesäurehexylester.

Dabei ist es erfindungsgemäß bevorzugt, wenn die Zubereitung 4-(tert.-Butyl)-4'-methoxydibenzoylmethan oder 2-(4'-(Diethylamino)-2'-hydoxybenzoyl)-benzoesäurehexylester enthält.

Enthält die erfindungsgemäße Zubereitung 4-(tert.-Butyl)-4'-methoxydibenzoylmethan, so ist es erfindungsgemäß vorteilhaft, wenn diese Verbindung in der Zubereitung in einer Menge von 0,1 bis 5 Gewichts-%, bezogen auf das Gesamtgewicht der Zubereitung eingesetzt wird. Erfindungsgemäß bevorzugt ist eine Einsatzkonzentration an 4-(tert.-Butyl)-4'-methoxydibenzoylmethan von 1 bis 5 Gewichts-%, bezogen auf das Gesamtgewicht der Zubereitung.

Enthält die erfindungsgemäße Zubereitung 2-(4'-(Diethylamino)-2'-hydoxybenzoyl)-benzoesäurehexylester, so ist es erfindungsgemäß vorteilhaft, wenn diese Verbindung in der Zubereitung in einer Menge von 0,1 bis 10 Gewichts-%, bezogen auf das Gesamtgewicht der Zubereitung eingesetzt wird. Erfindungsgemäß bevorzugt ist eine Einsatzkonzentration an 2-(4'-(Diethylamino)-2'-hydoxybenzoyl)-benzoesäurehexylester von 1 bis 8 Gewichts-%, bezogen auf das Gesamtgewicht der Zubereitung.

Werden Mischungen aus 4-(tert.-Butyl)-4'-methoxydibenzoylmethan und 2-(4'-(Diethylamino)-2'-hydoxybenzoyl)-benzoesäurehexylester eingesetzt, so beträgt die erfindungsgemäß vorteilhafte Gesamtkonzentration an beiden Substanzen von 1 bis 15 Gewichts-%, bezogen auf das Gesamtgewicht der Zubereitung, wobei eine Gesamtkonzentration von 3 bis 10 Gewichts-%, bezogen auf das Gesamtgewicht der Zubereitung erfindungsgemäß bevorzugt ist.

Es ist erfindungsgemäß vorteilhaft, wenn die erfindungsgemäße Zubereitung einen Lichtschutzfaktor (SPF) von mindestens 20 aufweist. Ein Lichtschutzfaktor von mindestens 30 ist erfindungsgemäß bevorzugt.

Erfindungsgemäß vorteilhaft, kann die erfindungsgemäße Zubereitung neben diesen erfindungswesentlichen UV-Filtern weitere UV-Filtersubstanzen enthalten.

So ist es erfindungsgemäß vorteilhaft, wenn die erfindungsgemäße Zubereitung 2-Phenylbenzimidazol-5-sulfonsäure und/oder deren Salze enthält.

Enthält die erfindungsgemäße Zubereitung 2-Phenylbenzimidazol-5-sulfonsäure und/oder deren Salze, so ist es erfindungsgemäß vorteilhaft, wenn diese in einer Gesamtkonzentration von 0,1 bis 10 Gewichts-%, bezogen auf das Gesamtgewicht der Zubereitung, in dieser enthalten sind. Erfindungsgemäß bevorzugt ist dabei der Konzentrationsbereich von 0,5 bis 2 Gewichts-%, bezogen auf das Gesamtgewicht der Zubereitung.

Es ist erfindungsgemäß vorteilhaft, wenn die erfindungsgemäße Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnltin, Carnosin, Coffein, natürliche und/oder synthetische Isoflavonoide, Glycerylglucose, Kreatin, Kreatinin, Taurin, ß-Alanin und/oder Licochalcon A, Panthenol, Tocopherol, Tocopherolacetat, Glycyrrhiza Inflata Root Extract, enthält.

Außerdem sind erfindungsgemäß vorteilhafte Ausführungsformen der vorliegenden Erfindung dadurch gekennzeichnet, dass die Zubereitung Propylenglycol, Butylenglycol, 2-Methylpropan-1,3-diol, Ethylhexylglycerin, 1,2-Pentandiol, 1,2-Hexandiol, 1,2-Octandiol und/oder 1,2-Decandiol enthält. Dabei ist der Gehalt an 2-Methylpropan-1,3-diol, 1,2-Pentandiol und/oder 1,2-Hexandiol erfindungsgemäß bevorzugt.

Es ist erfindungsgemäß vorteilhaft, wenn die erfindungsgemäße Zubereitung in Form einer Emulsion vorliegt und erfindungsgemäß bevorzugt, wenn die erfindungsgemäße Zubereitung in Form einer O/W-Emulsion vorliegt.

Liegt die erfindungsgemäße Zubereitung in Form einer O/W-Emulsion vor, so ist sie erfindungsgemäß vorteilhaft dadurch gekennzeichnet, dass die Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Natriumcetearylsulfat, Kaliumcetylphosphat, Polyglyceryl-10 Stearate Natriumstearylglutamat, enthält.

Diese erfindungsgemäßen O/W-Emulgatoren können erfindungsgemäß vorteilhaft in einer Konzentration von 0,001 bis 10 Gewichts-% und bevorzugt in einer Konzentration von 0,1 bis 7 Gewichts-%, bezogen auf das Gesamtgewicht der Zubereitung in dieser enthalten sein.

Die erfindungsgemäße Zubereitung kann vorteilhaft Feuchthaltemittel enthalten. Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben und/oder Aerosile® (CAS-Nr. 7631-86-9) und /oder Talkum, Polymethylsilsesquioxane, Nylon, Silica Dimethyl Silyate

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol oder Polyole niedriger C-Zahl sowie deren Ether, vorzugsweise Propylenglykol, Glycerin, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCI-Bezeichnung Acrylates/C10-30 Alkyl Acrylate Crosspolymer (z. B. Pemulen TR 1, Pemulen TR 2, Carbopol 1328 von der Fa. NOVEON) sowie Aristoflex AVC (INCI: Ammonium Acryloyldimethyltaurate/VP Copolymer).

Ein Gehalt an Glycerin von mindestens 5 Gewicht-%, bezogen auf das Gesamtgewicht der Zubereitung, ist dabei erfindungsgemäß besonders vorteilhaft.

Darüber hinaus ist es erfindungsgemäß besonders vorteilhaft, wenn die erfindungsgemäße Zubereitung C18-38 Alkyl Hydroxystearoyl Stearate und/oder Silica Dimethyl Silylate enthält

Erfindungsgemäß vorteilhaft enthält die erfindungsgemäße Zubereitung Filmbildner. Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im wesentlichen dazu dient, die Lichtfilter auf der Haut zu fixieren und so die Wasserfestigkeit des Produktes zu steigern.

Es ist insbesondere von Vorteil, die Filmbildner aus der Gruppe der Polymere auf Basis von Polyvinylpyrrolidon (PVP) 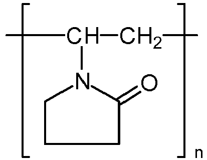 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole, Acrylat/Octylacralymid Copolymer (Dermacryl 79). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

Erfindungsgemäß ist die Verwendung der erfindungsgemäßen Zubereitung zum Schutz vor Hautalterung (insbesondere zum Schutz vor UV-bedingter Hautalterung) sowie als Sonnenschutzmittel.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

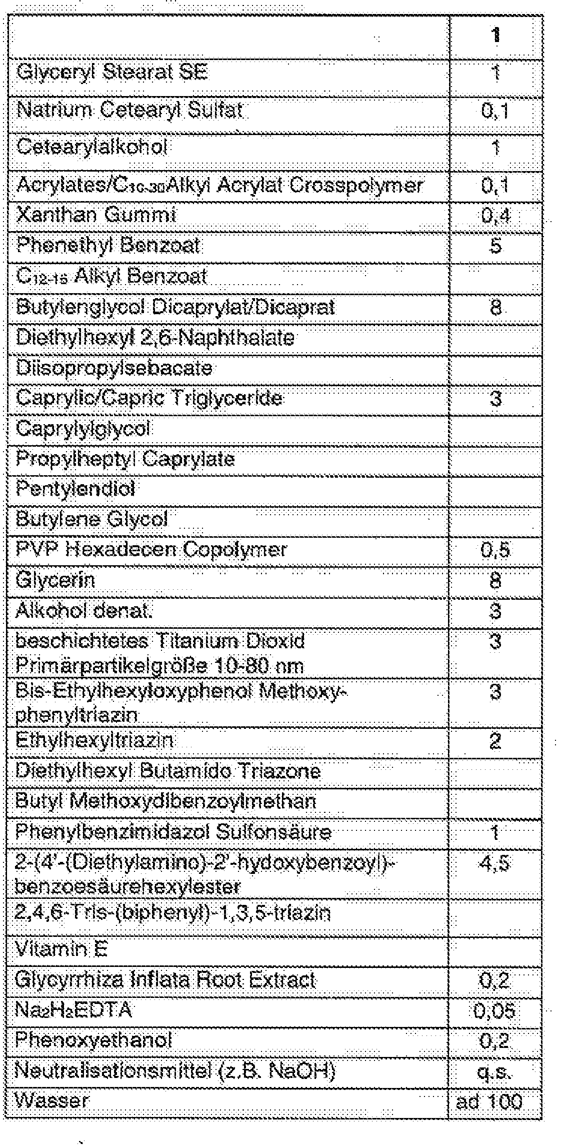

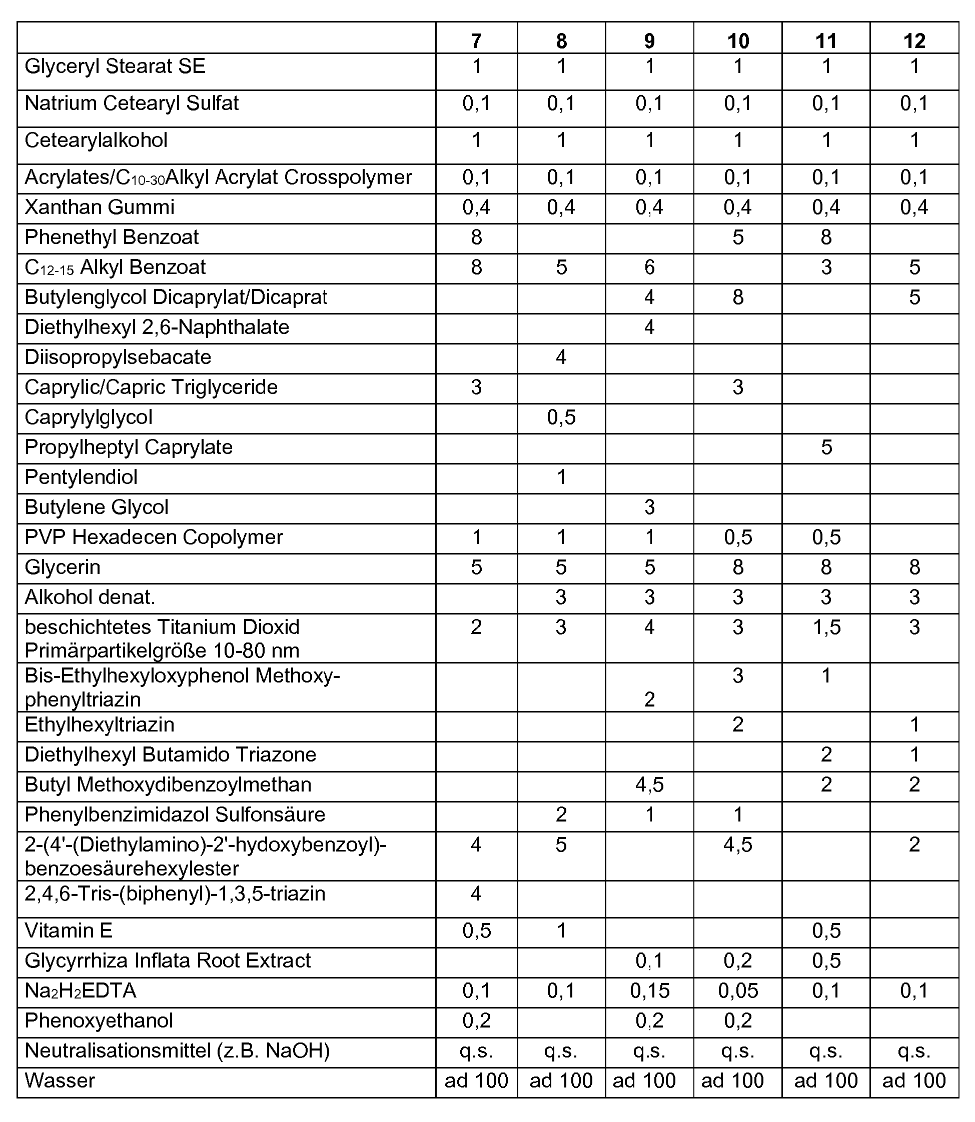

## Patentansprüche

1. Kosmetische Zubereitung enthaltend
a) ein oder mehrere UV-Filter aus der Gruppe der Triazinderivate,
b) Titandioxid,
c) 2-(4'-(Diethylamino)-2'-hydoxyberizoyl)-benzoosäurehexylester,
d) 2-Phenylbenzimidazol-5-sulfonsäure und/oder deren Salze,
e) ein oder mehrere Öle gewählt aus der Gruppe der Verbindungen Butylene Glycol Dicaprylat/Dicaprat, Phenethyl Benzoat, C12-15 Alkyl Benzoat, Dibutyladipat; Diisopropylbenzoate, Di-C12-13 Alkyl Tartrate, Butyloctyl Salicylate, Diethylhexyl Syringylidene Malonate, Hydragenated Castor Oil Dimerate, Triheptanoin, C12-13 Alkyl Lactate, C16-17 Alkyl Benzoate, Propylheptyl Caprylate, Caprylic/Capric Triglyceride, Diethylhexyl 2,6-Naphthalate, Octyldodecanol, Caprylic/Capric Triglyceride, Ethylhexyl Cocoate, wobei die Zubereitung frei ist von Ethylhexylsalicylat, 2-Ethylhexyl-2-cyano-3,3-diphenylacrylat, Homomenthylsalicylat, 4-Methoxyzimtsäure(2-ethylhexyl)ester und 3-(4-Methylbenzyliden)campher, Parabenen, Benzethonlumchlorid, Piroctone Olamine, Lauroylarginat, Benzoesäure, Sorbinsäure, Methylisothlazolinon, Chlormethylisothiazolinon, Bronopol, Benzalkoniumchloride, Formaldehydabspalter, Salicylsäure, Triclosan, Dehydroacetsäure , DMDM Hydantoin, Chlorphenesin, IPBC, **dadurch gekennzeichnet, dass** die Zubereitung Ethanol enthält.

2. Kosmetische Zubereitung nach Anspruch 1, **dadurch gekennzeichnet, dass** die Zubereitung 4-(tert-Butyl)-4'-methoxydibenzoylmethan enthält.

3. Kosmetische Zubereitung nach einem der vorhergehenden Ansprüche, **dadurch gekennzeichnet, dass** die Zubereitung frei ist von Polyethylenglycol, Polyethylenglycolethern und Polyethylenglycolestern.

4. Kosmetische Zubereitung nach einem der vorhergehenden Ansprüche, **dadurch gekennzeichnet, dass** die UV-Filter aus der Gruppe der Triazinderivate gewählt werden aus der Gruppe der Verbindungen Dioctylbutylamidotriazon (INCI: Diethylhexyl-Butamidotriazone); 2,4-Bis-{[4-(2-ethyl-hexyloxy)-2-hydroxy]-phenyl}-6-(4-methoxyphenyl)-1,3,5-triazin; 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin.

5. Kosmetische Zubereitung nach einem der vorhergehenden Ansprüche, **dadurch gekennzeichnet, dass** das Titandioxid eine Primärpartikelgröße von kleiner als 100 nm aufweist.

6. Kosmetische Zubereitung nach einem der vorhergehenden Ansprüche, **dadurch gekennzeichnet, dass** die Zubereitung UV-Filter aus der Gruppe der Triazinderivate In einer Gesamtmenge von 0,1 bis 10 Gewichts-%, bezogen auf das Gesamtgewicht der Zubereitung, enthält.

7. Kosmetische Zubereitung nach einem der vorhergehenden Ansprüche, **dadurch gekennzeichnet**, das die Zubereitung Titandioxid in einer Gesamtmenge von 0,5 bis 20 Gewichts-%, bezogen auf das Gesamtgewicht der Zubereitung, enthält.

8. Kosmetische Zubereitung nach einem der vorhergehenden Ansprüche, **dadurch gekennzeichnet, dass** die Zubereitung einen Lichtschutzfaktor (SPF) von mindestens 20 aufweist.

9. Kosmetische Zubereitung nach einem der vorhergehenden Ansprüche, **dadurch gekennzeichnet, dass** die Zubereitung zwei unterschiedliche Titandioxide enthält, die sich in ihrer Primärpartikelgröße voneinander unterscheiden.

10. Kosmetische Zubereitung nach einem der vorhergehenden Ansprüche, **dadurch gekennzeichnet, dass** die Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, ß-Alanin und/oder Licochalcon A, Panthenol, Tocopherol, Tocopherolacetat, Glycyrrhiza Inflata Root Extract enthält.

11. Kosmetische Zubereitung nach einem der vorhergehenden Ansprüche, **dadurch gekennzeichnet, dass** die Zubereitung Propylenglycol, Butylenglycol, 2-Methylpropan-1,3-diol, 1,2-Pentandiol, 1,2-Hexandiol, 1,2-Octandiol und/oder 1,2-Decandiol enthält.

12. Kosmetische Zubereitung nach einem der vorhergehenden Ansprüche, **dadurch gekennzeichnet, dass** die Zubereitung in Form einer O/W-Emulsion vorliegt.

## Claims

1. Cosmetic preparation comprising
a) one or more UV filters from the group of triazine derivatives,
b) titanium dioxide,
c) hexyl 2-(4'-(diethylamino)-2'-hydroxybenzoyl)benzoate,
d) 2-phenylbenzimidazole-5-sulfonic acid and/or salts thereof,
e) one or more oils selected from the group of compounds comprising butylene glycol dicaprylate/dicaprate, phenethyl benzoate, C12-15 alkyl benzoate, dibutyl adipate; diisopropyl benzoate, di-C12-13 alkyl tartrate, butyloctyl salicylate, diethylhexyl syringylidenemalonate, hydrogenated castor oil dimerate, triheptanoin, C12-13 alkyl lactate, C16-17 alkyl benzoate, propylheptyl caprylate, caprylic/capric triglyceride, diethylhexyl 2,6-naphthalate, octyldodecanol, caprylic/capric triglyceride, ethylhexyl cocoate, wherein the preparation is free from ethylhexyl salicylate, 2-ethylhexyl 2-cyano-3,3-diphenylacrylate, homomenthyl salicylate, 2-ethylhexyl 4-methoxycinnamate and 3-(4-methylbenzylidene)camphor, parabens, benzethonium chloride, piroctone olamine, lauroyl arginate, benzoic acid, sorbic acid, methylisothiazolinone, chloromethylisothiazolinone, bronopol, benzalkonium chloride, formaldehyde releasers, salicylic acid, triclosan, dehydroacetic acid, DMDM hydantoin, chlorphenesin, IPBC, **characterized in that** the preparation comprises ethanol.

2. Cosmetic preparation according to Claim 1, **characterized in that** the preparation comprises 4-(tert-butyl)-4'-methoxydibenzoylmethane.

3. Cosmetic preparation according to either of the preceding claims, **characterized in that** the preparation is free from polyethylene glycol, polyethylene glycol ethers and polyethylene glycol esters.

4. Cosmetic preparation according to any of the preceding claims, **characterized in that** the UV filters from the group of triazine derivatives are selected from the group of compounds comprising dioctylbutylamidotriazone (INCI: Diethylhexyl Butamido Triazone), 2,4-bis-{[4-(2-ethylhexyloxy)-2-hydroxy]phenyl}-6-(4-methoxyphenyl)-1,3,5-triazine; tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4,6-tribiphenyl-4-yl-1,3,5-triazine.

5. Cosmetic preparation according to any of the preceding claims, **characterized in that** the titanium dioxide has a primary particle size of less than 100 nm.

6. Cosmetic preparation according to any of the preceding claims, **characterized in that** the preparation comprises UV filters from the group of triazine derivatives in a total amount of 0.1 to 10% by weight, based on the total weight of the preparation.

7. Cosmetic preparation according to any of the preceding claims, **characterized in that** the preparation comprises titanium dioxide in a total amount of 0.5 to 20% by weight, based on the total weight of the preparation.

8. Cosmetic preparation according to any of the preceding claims, **characterized in that** the preparation has a sun protection factor (SPF) of at least 20.

9. Cosmetic preparation according to any of the preceding claims, **characterized in that** the preparation comprises two different titanium dioxides which differ from each other in their primary particle size.

10. Cosmetic preparation according to any of the preceding claims, **characterized in that** the preparation comprises one or more active ingredients selected from the group of compounds comprising gylcyrrhetic acid, urea, arctiin, alpha-lipoic acid, folic acid, phytoene, D-biotin, coenzyme Q10, alpha-glucosylrutin, carnitine, carnosine, caffeine, natural and/or synthetic isoflavonoids, glycerylglucose, creatine, creatinine, taurine, β-alanine and/or licochalcone A, panthenol, tocopherol, tocopherol acetate, Glycyrrhiza inflata root extract.

11. Cosmetic preparation according to any of the preceding claims, **characterized in that** the preparation comprises propylene glycol, butylene glycol, 2-methylpropane-1,3-diol, 1,2-pentanediol, 1,2-hexanediol, 1,2-octanediol and/or 1,2-decanediol.

12. Cosmetic preparation according to any of the preceding claims, **characterized in that** the preparation is in the form of an O/W emulsion.

## Revendications

1. Préparation cosmétique contenant
a) un ou plusieurs filtres UV du groupe des dérivés de triazine,
b) du dioxyde de titane,
c) de l'ester d'hexyle de l'acide 2-(4'-(diéthylamino)-2'-hydoxybenzoyl)-benzoïque,
d) de l'acide 2-phénylbenzimidazol-5-sulfonique et/ou ses sels,
e) une ou plusieurs huiles choisies dans le groupe des composés dicaprylate/dicaprate de butylèneglycol, benzoate de phénéthyle, benzoate de C₁₂₋₁₅-alkyle, adipate de dibutyle ; benzoate de diisopropyle, tartrates de di-C₁₂₋₁₃-alkyle, salicylate de butyloctyle, syringylidène malonate de diéthylhexyle, dimérate d'huile de castor hydrogénée, triheptanoïne, lactate de C₁₂₋₁₃-alkyle, benzoate de C₁₆₋₁₇-alkyle, caprylate de propylheptyle, triglycéride caprylique/caprique, 2,6-naphtalate de diéthylhexyle, octyldodécanol, triglycéride caprylique/caprique, cocoate d'éthylhexyle, la préparation étant exempte de salicylate d'éthylhexyl, 2-éthylhexyl-2-cyano-3,3-diphénylacrylate, salicylate d'homomenthyle, ester de 2-éthylhexyle d'acide 4-méthoxycinnamique et 3-(4-méthylbenzylidène)camphre, parabènes, chlorure de benzéthonium, piroctone olamine, arginate de lauroyle, acide benzoïque, acide sorbique, méthylisothiazolinone, chlorométhylisothiazolinone, bronopol, chlorure de benzalkonium, agents libérant du formaldéhyde, acide salicylique, triclosan, acide déhydro-acétique, DMDM hydantoïne, chlorophénésine, IPBC, **caractérisée en ce que** la préparation contient de l'éthanol.

2. Préparation cosmétique selon la revendication 1, **caractérisée en ce que** la préparation contient du 4-(tert-butyl)-4'-méthoxydibenzoylméthane.

3. Préparation cosmétique selon l'une quelconque des revendications précédentes, **caractérisée en ce que** la préparation est exempte de polyéthylène glycol, d'éthers de polyéthylène glycol et d'esters de polyéthylène glycol.

4. Préparation cosmétique selon l'une quelconque des revendications précédentes, **caractérisée en ce que** les filtres UV sont choisis dans le groupe des dérivés de triazine du groupe de composés dioctylbutylamidotriazone (INCI : Diethylhexyl-Butamidotriazone) ; 2,4-bis-{[4-(2-éthyl-hexyloxy)-2-hydroxy]-phényl}-6-(4-méthoxyphényl)-1,3,5-triazine ; triester de 2-éthylhexyle d'acide 4,4',4"-(1,3,5-triazin-2,4,6-triyltriimino)-tris-benzoïque (également : 2,4,6-tris-[anilino-(p-carbo-2'-éthyl-1'-hexyloxy)]-1,3,5-triazine (INCI : Ethylhexyl Triazone) ; 2,4,6-tribiphényl-4-yl-1,3,5-triazine.

5. Préparation cosmétique selon l'une quelconque des revendications précédentes, **caractérisée en ce que** le dioxyde de titane présente une grosseur de particule primaire inférieure à 100 nm.

6. Préparation cosmétique selon l'une quelconque des revendications précédentes, **caractérisée en ce que** la préparation contient des filtres UV du groupe des dérivés de triazine en une quantité totale de 0,1 à 10 % en poids, par rapport au poids total de la préparation.

7. Préparation cosmétique selon l'une quelconque des revendications précédentes, **caractérisée en ce que** la préparation contient du dioxyde de titane en une quantité totale de 0,5 à 20 % en poids, par rapport au poids total de la préparation.

8. Préparation cosmétique selon l'une quelconque des revendications précédentes, **caractérisée en ce que** la préparation présente un facteur de protection solaire (SPF) d'au moins 20.

9. Préparation cosmétique selon l'une quelconque des revendications précédentes, **caractérisée en ce que** la préparation contient deux dioxydes de titane différents, qui se différencient l'un de l'autre par leur grosseur de particule primaire.

10. Préparation cosmétique selon l'une quelconque des revendications précédentes, **caractérisée en ce que** la préparation contient un ou plusieurs principes actifs choisis dans le groupe des composés acide glycyrrhétinique, urée, arctiine, acide alpha-lipoïque, acide folique, phytoène, D-biotine, coenzyme Q10, alpha-glucosylrutine, carnitine, carnosine, caféine, isoflavonoïdes naturels et/ou synthétiques, glycérylglucose, créatine, créatinine, taurine, β-alanine et/ou licochalcone A, panthénol, tocophérol, acétate de tocophérol, extrait de racine de Glycyrrhiza Inflata.

11. Préparation cosmétique selon l'une quelconque des revendications précédentes, **caractérisée en ce que** la préparation contient du propylèneglycol, du butylèneglycol, du 2-méthylpropane-1,3-diol, du 1,2-pentanediol, du 1,2-hexanediol, du 1,2-octanediol et/ou du 1,2-décanediol.

12. Préparation cosmétique selon l'une quelconque des revendications précédentes, **caractérisée en ce que** la préparation est présente sous forme d'une émulsion huile/eau.
